# EUROPÄISCHE PATENTSCHRIFT

(11) **EP 2 742 958 B1**
(45) Veröffentlichungstag und Bekanntmachung des Hinweises auf die Patenterteilung: **09.09.2015**
(21) Anmeldenummer: 13196192.2
(22) Anmeldetag: 09.12.2013
(51) Int. Cl.: A61L 2/08, B29C 49/42, B65B 55/02, B67C 7/00

(54) **Vorrichtung zum Sterilisieren von Behältnissen**
Apparatus for sterilising containers
Appareil pout steriliser des récipients

(30) Priorität: 12.12.2012 DE 102012112158
(43) Veröffentlichungstag der Anmeldung: 18.06.2014
(73) Patentinhaber: Krones AG, 93073 Neutraubling (DE)
(72) Erfinder: Voth, Klaus, 93073 Neutraubling (DE); Laumer, Roland, 93073 Neutraubling (DE); Hüttner, Gerald, 93073 Neutraubling (DE); Söllner, Jürgen, 93073 Neutraubling (DE); Loy, Michael, 93073 Neutraubling (DE)
(74) Vertreter: Bittner, Bernhard

(56) Entgegenhaltungen:
- EP-A1- 2 213 578
- EP-A1- 2 371 397
- EP-A1- 2 394 950
- EP-A1- 2 594 495
- EP-A1- 2 604 410
- EP-A1- 2 650 022
- WO-A1-99/64220

## Beschreibung

Die Erfindung betrifft eine Vorrichtung zum Sterilisieren von Behältnissen, insbesondere von Kunststoffbehältnissen und insbesondere von Kunststoffvorformlingen sowie ein Verfahren zum Sterilisieren von Behältnissen, insbesondere von Kunststoffbehältnissen und insbesondere von Kunststoffvorformlingen. Es wird darauf hingewiesen, dass die Erfindung im Folgenden unter Bezugnahme auf Kunststoffvorformlinge beschrieben wird und auch lediglich von Kunststoffvorformlingen die Rede sein wird. Die Erfindung ist jedoch entsprechend auch auf die Sterilisation von Behältnissen und insbesondere von Kunststoffbehältnissen anwendbar. Die Erfindung eignet sich jedoch in besonderem Maße für Kunststoffvorformlinge, da diese ein relativ geringes Gewicht aufweisen und auch im Hinblick auf ihre Größe relativ einfach sterilisierbar sind.

Die Vorrichtung zum Sterilisieren von Kunststoffvorformlingen umfasst eine Sterilisationskammer und eine zumindest teilweise innerhalb der Sterilisationskammer angeordnete Transporteinrichtung zum Bewegen der Kunststoffvorformlinge innerhalb der Sterilisationskammer, wobei die Transporteinrichtung zumindest ein Greifsystem zum Umgreifen und Tragen der Kunststoffvorformlinge mittels einer Greifklammer aufweist, gemäß dem Oberbegriff des Patentanspruches 1. Unter einem Umgreifen wird dabei ein zumindest teilweises Umgreifen verstanden, es ist im Rahmen der Erfindung also nicht unbedingt erforderlich, dass der Kunststoffvorformling in seiner Umfangsrichtung vollständig umgriffen wird.

Gattungsgemäße Vorrichtungen zum Sterilisieren von Kunststoffvorformlingen sind bereits aus dem Stand der Technik bekannt. Dabei werden die Kunststoffvorformlinge durch eine Sterilisationskammer mittels der Transporteinrichtung hindurchgeführt und während des Hindurchführens beispielsweise mittels eines Sterilisationsmittels sterilisiert. Um die Haltbarkeit von sensiblen Füllprodukten beispielsweise in PET-Flaschen entscheidend zu verbessern, muss vor dem Füllprozess die Anzahl der Keime in den Behältern deutlich reduziert werden. Dazu sind im Bereich der Fülltechnik verschiedene Nass- und Trockenaseptikmethoden bekannt, die jedoch aufgrund der teilweise großen Flaschenvolumina einen hohen Verbrauch an verwendetem Sterilisationsmedium (z.B. Peressigsäure, H₂O₂, ...) bedingen. Weitere Maschinenkonzepte reduzieren deshalb die Anzahl der Keime bereits vor dem Blasen der Kunststoffvorformlinge. Dabei durchläuft der Kunststoffvorformling einen Behandlungsbereich, also eine Sterilisationskammer, in dem die Entkeimung durch gasförmige oder flüssige Sterilisationsmedien oder durch Bestrahlung (UV-Strahlung, Elektronenstrahlen) erzielt wird.

Die EP 2604410 A1 beschreibt eine Vorrichtung zum Behandeln von Kunststoffbehältnissen, wobei eine Zuführeinrichtung Heizstreckentransportmittel zum Transportieren der Kunststoffvorformlinge entlang einer Heizstrecke aufweist, um die Kunststoffvorformlinge mindestens einer Außenentkeimungseinheit zuzuführen.

Die EP 2371317 beschreibt ebenfalls eine Vorrichtung zum Sterilisieren von Behältnissen. Dabei weist die Vorrichtung eine erste und eine zweite Sterilisationseinrichtung zur Sterilisation einer Außenwandung der Behältnisse auf.

Aus der EP 213578 ist eine Vorrichtung zum Sterilisieren von Behältnissen bekannt. Dabei werden zur Sterilisation Elektronenstrahlen verwendet.

Die EP 2594495 beschreibt ebenfalls eine Vorrichtung zur Behältnissterilisation. Dabei ist ein Haltelement zum Halten des Behältnisses über die Mündung in das Behältnis einführbar.

Aus der WO 99/64220 A1 ist ein Verfahren und eine Vorrichtung zum Herstellen von Hohlkörpern bekannt. Dabei werden die Kunststoffvorformlinge mittels eines Shuttlesystems gefördert.

Die EP 2304950 A1 beschreibt ein Verfahren zum Befüllen von Behältnissen.

Aus dem Stand der Technik bekannte Vorrichtungen zum Sterilisieren der Kunststoffvorformlinge tragen jedoch den Nachteil in sich, besonders hohen Anforderungen an die Entkeimungsleistung (z.B. bei schwach sauren Füllprodukten) nicht entsprechen zu können. Generell sollte nämlich bei der Behälterbehandlung sichergestellt werden, dass jeder Bereich des Behälters für eine bestimmte Zeit mit dem Sterilisationsmittel, das heißt einem Sterilisationsmedium bzw. der Bestrahlung, in Berührung kommt. Eine derart möglichst umfassende und vollflächige Sterilisation ist insbesondere im Falle einer späteren Befüllung mit schwach sauren Füllprodukten wünschenswert. Ein Problem stellt hierbei das Greifsystem dar, das an den Stellen einer kraft- bzw. formschlüssigen Verbindung einer Greifklammer mit dem Kunststoffvorformling, also an Kontaktflächen zwischen der Greifklammer und dem Kunststoffvorformling, nicht oder nur in unzureichendem Maße von dem Sterilisationsmittel sterilisiert werden kann. Mit anderen Worten werden derartige Kontaktflächen auf einer Außenfläche des Kunststoffvorformlings von der Greifklammer verdeckt. Eine Entkeimung dieser Kontaktflächen auf dieser Außenfläche wird dadurch nicht sichergestellt oder zumindest erschwert, was insbesondere im füllproduktnahen Mündungsbereich problematisch ist.

Eine zu lösende Aufgabe besteht daher darin, eine kostengünstige Vorrichtung zum Sterilisieren von Kunststoffvorformlingen anzubieten, bei denen eine Außenfläche von Kunststoffvorformlingen in möglichst einfacher Art und Weise vollständig sterilisierbar ist.

Dies wird erfindungsgemäß durch eine Vorrichtung zum Sterilisieren von Kunststoffvorformlingen nach Anspruch 1 und ein Verfahren zum Sterilisieren von Kunststoffvorformlingen nach Anspruch 7 erreicht. Vorteilhafte Ausführungsformen und Weiterbildungen sind Gegenstand der Unteransprüche.

Um nun eine Vorrichtung zum Sterilisieren von Kunststoffvorformlingen anzubieten, bei denen in besonders einfacher und kostengünstiger Weise eine Außenfläche der Kunststoffvorformlinge vollständig sterilisierbar ist, macht die vorliegende Erfindung unter anderem von der Idee Gebrauch, dass das Greifsystem ein Verlagerungsmittel zur Verlagerung während eines Sterilisierens innerhalb der Sterilisationskammer von, durch das Greifsystem vor einem Sterilisationsmedium und/oder einer Sterilisationsstrahlung abgeschatteten, Kontaktflächen auf einer Außenfläche der Kunststoffvorformlinge aufweist. Wird ein Kunststoffvorformling mittels der Greifklammer über die Transporteinrichtung durch die Sterilisationskammer hindurchgeführt, ist denkbar, dass nach einer vorgebbaren Teilsterilisationszeit das Verlagerungsmittel während des Zugreifens des Greifsystems, beispielsweise mittels eines kraftschlüssigen Kontaktes, zwischen dem Verlagerungsmittel und dem Kunststoffvorformling, das Verlagerungsmittel den Kunststoffvorformling innerhalb des Greifsystems verdreht und/oder verschiebt. Kontaktflächen auf der Außenfläche des Kunststoffvorformlings, welche zu Beginn der Sterilisation noch von dem Greifsystem für eine Sterilisation verdeckt waren, sind nach dem beispielsweise Verdrehen des Kunststoffvorformlings durch das Verlagerungsmittel nun frei und damit für die restlich verbleibende Zeit der Sterilisation auch vollständig sterilisierbar. Ein derartiges Verlagerungsmittel ermöglicht daher ein möglichst umfassendes und vollständiges Sterilisieren der gesamten Außenfläche des Kunststoffvorformlings, ohne dass der Sterilisationsvorgang unterbrochen werden müsste oder mittels weiterer aufwändiger Umgreif- oder Verschiebemechaniken der Kunststoffvorformling verlagert werden müsste.

Gemäß zumindest einer Ausführungsform umfasst die Vorrichtung zum Sterilisieren von Kunststoffvorformlingen eine Sterilisationskammer und eine zumindest teilweise innerhalb der Sterilisationskammer angeordnete Transporteinrichtung zum Bewegen der Kunststoffvorformlinge innerhalb der Sterilisationskammer, wobei die Transporteinrichtung zumindest ein Greifsystem zum Umgreifen und Tragen der Kunststoffvorformlinge mittels einer Greifklammer aufweist. Das Greifsystem weist ein Verlagerungsmittel zur Verlagerung während eines Sterilisierens innerhalb der Sterilisationskammer von, durch das Greifsystem vor einem Sterilisationsmedium abgeschatteten, Kontaktflächen auf einer Außenfläche der Kunststoffvorformlinge auf.

Bevorzugt weist die Vorrichtung wenigstens eine Beaufschlagungseinrichtung auf, welche die Kunststoffvorformlinge mit einem fließfähigen Sterilisationsmedium (beispielsweise Wasserstoffperoxid und/oder Peressigsäure) beaufschlagt und/oder eine Strahlungseinrichtung, welche die Kunststoffvorformlinge mit einer Strahlung, insbesondere einer elektromagnetischen Strahlung bzw. mit Ladungsträgern, insbesondere mit Elektronen (denkbar wären jedoch auch etwa Protonen oder alpha-Teilchen) beaufschlagt. Vorteilhaft ist dabei eine Beaufschlagungseinrichtung und/oder eine Strahlungseinrichtung stationär angeordnet.

Gemäß zumindest einer Ausführungsform ist das Verlagerungsmittel mit zumindest einer Antriebsrolle gebildet, welche drehbar innerhalb der Greifklammer angeordnet ist und kraftschlüssig mit dem Kunststoffvorformling in Verbindung steht, und wobei über eine Rotation der Antriebsrolle um deren Rotationsachse, relativ zu Greifarmen der Greifklammer, in eine Drehrichtung, eine Rotation des Kunststoffvorformlings um dessen Rotationsachse, relativ zu den Greifarmen, bewirkbar ist, wobei beide Rotationsrichtungen jeweils entgegengesetzt sind. Das heißt, dass der Kunststoffvorformling innerhalb der Greifklammer während des Umgreifens und Tragens durch die Greifklammer mittels der Antriebsrolle gedreht wird. Beispielsweise zu Beginn eines Sterilisationsvorganges innerhalb der Sterilisationskammer durch die Greifarme für das Sterilisationsmittel verdeckte Stellen der Außenfläche des Kunststoffvorformlings werden daher durch das Verdrehen für das Sterilisationsmedium freigelegt. Dadurch wird besonders einfach sichergestellt, dass alle Außenflächenbereiche des Kunststoffvorformlings eine ausreichende Zeit mit dem Sterilisationsmittel, beispielsweise einem Sterilisationsmedium oder einer Bestrahlung, in Berührung kommen.

Gemäß zumindest einer Ausführungsform ist die Antriebsrolle mittels eines pneumatischen, mechanischen, elektrischen oder hydraulischen Antriebes rotierbar. Dazu kann der Antrieb von dem Greifsystem umfasst sein oder der Antrieb getrennt von dem Greifsystem mechanisch fest an der Transporteinrichtung und/oder innerhalb der Sterilisationskammer angeordnet sein.

Gemäß zumindest einer Ausführungsform ist das Verlagerungsmittel mit zumindest einer Reibschiene gebildet, wobei der Kunststoffvorformling innerhalb der Sterilisationskammer entlang dieser Reibschiene vorbeiführbar ist, wobei über eine kraftschlüssige Verbindung zwischen dem Kunststoffvorformling und der Reibschiene der Kunststoffvorformling innerhalb der Greifklammer, also während des Umgreifens und Tragens, um seine Rotationsachse drehbar ist. Die Kunststoffvorformlinge werden daher an der Reibschiene vorbeigeführt, was unmittelbar zu einer Drehung der Kunststoffvorformlinge innerhalb der Greifarme der Greifklammer führt.

Je länger die Reibschiene ausgebildet ist, desto länger sind die Kunststoffvorformlinge in kraftschlüssigem Kontakt mit dieser Reibschiene und werden entsprechend der Länge der Reibschiene und über vorzugsweise die gesamte Strecke der Länge der Reibschiene gedreht. Mit anderen Worten bestimmt die Länge der Reibschiene einen Verdrehwinkel der Kunststoffvorformlinge ausgehend von der Ausgangstellung. Mittels einer derartigen Reibschiene ist besonders einfach ein Einstellen des Verdrehwinkels ermöglicht. Denkbar ist, dass zur Einstellung der Verdrehwinkels verschiedene Reibschienen in der Vorrichtung vorgehalten werden und je nach Bedarf und Ausbildung der Greifarme, welche eine Flächenabmessung der Kontaktflächen zwischen den Greifarmen und den Kunststoffvorformlingen bestimmen, die Länge der Reibschiene ausgewählt wird. Alternativ ist die Länge einer Reibschiene veränderbar, beispielsweise teleskopierbar.

Gemäß der Erfindung ist das Verlagerungsmittel mit zumindest einer weiteren Greifklammer gebildet, wobei beide Greifklammern in vertikaler Richtung zumindest teilweise überlappend und in dieser Richtung beabstandet zueinander angeordnet sind. "Vertikale Richtung" ist eine Richtung senkrecht zu einer Transportebene der Transporteinrichtung. Dabei ist während des Sterilisierens zumindest eine Greifklammer in einer Offen-Stellung und eine davon verschiedene Greifklammer in einer Zu-Stellung betreibbar, wobei nach einer vorgebbaren Teil-Sterilisationszeit die jeweils andere Greifklammer in einer Offen-Stellung oder in einer Zu-Stellung betreibbar ist.

Mit anderen Worten ist während des Sterilisierens innerhalb der Sterilisationskammer zumindest eine Greifklammer geschlossen und greift kraftschlüssig den Kunststoffvorformling. Der Kunststoffvorformling ist daher während des Sterilisierens durch zumindest eine Greifklammer stets fest umgriffen und gehalten. Ist das Verlagerungsmittel nun durch beispielsweise zwei Greifklammern gebildet, ist die jeweils andere Greifklammer in der Offen-Stellung, das heißt diese Greifklammer ist vorzugsweise nicht in Kontakt, insbesondere nicht in kraftschlüssigem Kontakt, mit dem Kunststoffvorformling.

Nach Ablauf der vorgebbaren Teil-Sterilisationszeit greift nun die bisher in der Offen-Stellung betriebene weitere Greifklammer mittels ihrer Greifarme den Kunststoffvorformling. Unmittelbar nach dem Zugreifen der weiteren Greifklammer öffnet die bis zu diesem Zeitpunkt noch geschlossene Greifklammer und wird in die Offen-Stellung gefahren. Mit anderen Worten ist nach Ablauf der Teilsterilisationszeit für die restlich verbleibende Sterilisationszeit nunmehr die weitere Greifklammer in der Zu-Stellung und umgreift den Kunststoffvorformling kraftschlüssig, während die Greifklammer nun folglich nicht mehr in direktem, insbesondere nicht in kraftschlüssigem Kontakt, mit dem Kunststoffvorformling ist.

Jeweilige Kontaktflächen auf der Außenfläche des Kunststoffvorformlings, zwischen dem Kunststoffvorformling und den Greifarmen der Greifklammern, werden daher nach dem jeweiligen Ablauf der Teil-Sterilisationszeit verlagert. Vormals bis zum Ablauf der TeilSterilisationszeit definierte Kontaktflächen sind daher nach Ablauf der Teil-Sterilisationszeit dem Sterilisationsmittel frei zugänglich, während durch das Zugreifen der weiteren Greifklammer mittels ihrer Greifarme dadurch verschobene Kontaktflächen, welche bereits sterilisiert sind, nunmehr für das Sterilisationsmittel abgeschattet sind.

Gemäß zumindest einer Ausführungsform sind Klammergelenke der Greifklammern in vertikaler Richtung übereinander angeordnet, wobei jeweilige Klammeröffnungen der beiden Greifklammern in der vertikalen Richtung zumindest teilweise überlappen. Dabei sind die Klammergelenke der Greifklammer diejenigen Gelenke, in denen die den jeweiligen Greifklammern zugeordneten Greifarme drehbar und gemeinsam gelagert sind. Klammeröffnungen sind diejenigen den Klammergelenken gegenüberliegenden Bereiche der Greifklammer, welche in der der Zu-Stellung der Greifklammer frei von den Greifarmen ist. Die Klammeröffnungen bilden daher eine zwischen den Greifklammern ausgebildete Lücke aus.

Vorzugsweise sind beide Greifklammern gemeinsam an der Transporteinrichtung mechanisch befestigt. Mit anderen Worten umfasst das Greifsystem in diesem Fall zumindest zwei Greifarmpaare zweier Greifklammern, wobei beide Greifklammern fest an der Transporteinrichtung montiert sind. Ein in diesem Ausführungsbeispiel beschriebenes Umgreifen, das heißt Verlagern der Kontaktflächen, kann daher durch ein und dieselbe Transporteinrichtung besonders einfach realisiert werden.

Gemäß zumindest einer Ausführungsform sind die Greifklammern an verschiedenen, sich jeweils in horizontaler Richtung gegenüberliegenden Klammergelenken angeordnet, wobei das Klammergelenk der weiteren Greifklammer an einer weiteren Transporteinrichtung, welche zumindest teilweise innerhalb der Sterilisationskammer angeordnet ist, befestigt ist. "Horizontale Richtung" ist eine Richtung parallel zur Transportebene der Transporteinrichtung. Vorzugsweise sind beide Transporteinrichtungen vollständig innerhalb der Sterilisationskammer angeordnet. Ein Umgreifen, das heißt eine Verlagerung der Kontaktflächen zwischen den Greifklammern und dem Kunststoffvorformling findet daher zwischen diesen beiden Greifklammern, welche separat und getrennt voneinander angeordnet sind, statt.

Dabei übergibt die Greifklammer, welche bevorzugt auf und bevorzugt mechanisch fest mit dem Transportsystem mechanisch verbunden ist, den Kunststoffvorformling an die weitere Greifklammer, welche bevorzugt auf und bevorzugt mechanisch fest an dem weiteren Transportsystem befestigt ist. Insofern wird nach Ablauf der Teilsterilisationszeit der von der Greifklammer festgehaltene Kunststoffvorformling an die weitere Greifklammer übergeben. Nach dem kraftschlüssigen Zugreifen der weiteren Greifklammer um den Kunststoffvorformling herum löst sich dann die Greifklammer, so dass während der Übergabe für einen vorgebbaren Zeitraum, welcher vorzugsweise besonders kurz ausgestaltet ist, beide Greifklammern in der Zu-Stellung betrieben sind und daher während einer so definierten Übergabezeit den Kunststoffvorformling kraftschlüssig umgreifen.

Vorteilhaft wird jedoch der Sterilisationsvorgang auch während dieses Umgreifens fortgesetzt.

Es wird darüber hinaus ein Verfahren zum Sterilisieren von Behältnissen und insbesondere von Kunststoffvorformlingen angegeben. Mittels des hier beschriebenen Verfahrens kann ein Kunststoffvorformling sterilisiert werden, wie es in Verbindung mit einem oder mehreren der oben genannten Ausführungsformen beschrieben ist. Das heißt, die für die hier beschriebene Vorrichtung aufgeführten Merkmale sind auch für das hier beschriebene Verfahren offenbart und umgekehrt.

In dem hier beschriebenen Verfahren werden über eine zumindest teilweise innerhalb einer Sterilisationskammer angeordnete Transporteinrichtung Kunststoffvorformlinge bewegt, wobei die Transporteinrichtung zumindest ein Greifsystem zum Umgreifen und Tragen der Kunststoffvorformlinge mittels einer Greifklammer aufweist.

Während eines Sterilisierens innerhalb der Sterilisationskammer, nach Ablauf einer vorgebbaren Teilsterilisationszeit, werden mittels eines Verlagerungsmittels durch das Greifsystem von einem Sterilisationsmedium und/oder einer Sterilisationsstrahlung abgeschattete Kontaktflächen der Kunststoffvorformlinge verlagert. Wie bereits eingangs beschrieben, ermöglicht ein derartiges Verlagern der Kontaktflächen, welche zwischen Greifarmen der Greifklammer und dem Kunststoffvorformling ausgebildet sind möglichst vollständiges Sterilisieren der gesamten Oberfläche des Kunststoffvorformlings. Dieses Verlagern kann beispielsweise mittels eines Rotierens innerhalb der Greifklammer des Kunststoffvorformlings geschehen oder aber mittels eines Umgreifens zwischen der Greifklammer und zumindest einer weiteren Greifklammer, wodurch ebenso die jeweiligen Kontaktflächen der Kunststoffvorformlinge verlagert werden.

Auch kann dieses Verlagern durch ein Verschieben des Kunststoffvorformlings in seiner Längsrichtung gegenüber dem Greifelement erfolgen.

Im Folgenden werden die hier beschriebene Vorrichtung sowie das hier beschriebene Verfahren anhand von Ausführungsbeispielen und den dazugehörigen Figuren näher erläutert.

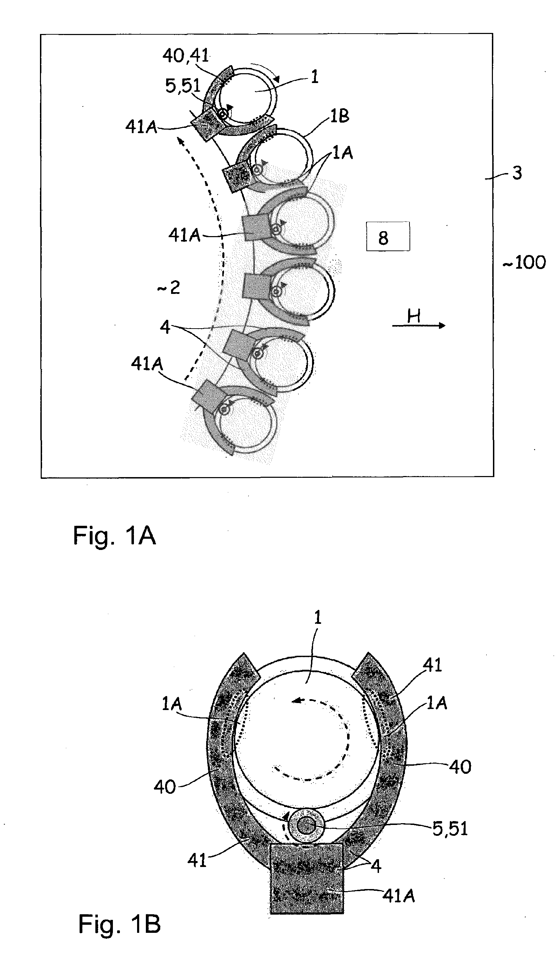

Die Figuren 1A bis1G zeigen in verschiedenen, schematisch perspektivischen Ansichten Ausführungsbeispiele einer hier beschriebenen Vorrichtung sowie Verfahrensschritte zum Sterilisieren von Kunststoffvorformlingen.

In den Ausführungsbeispielen und in den Figuren sind gleiche oder gleichwirkende Bestandteile jeweils mit den gleichen Bezugszeichen versehen. Die dargestellten Elemente sind nicht als maßstabgerecht anzusehen, vielmehr können einzelne Elemente zum besseren Verständnis übertrieben groß dargestellt sein.

In der Figur 1A ist anhand einer schematischen Draufsicht eine hier beschriebene Vorrichtung 100 zum Sterilisieren von Kunststoffvorformlingen 1 gezeigt. Die Vorrichtung 100 weist eine Sterilisationskammer 3 und eine vollständig innerhalb der Sterilisationskammer 3 angeordnete (insb. drehbare) Transporteinrichtung 2 zum Bewegen der Kunststoffvorformlinge 1 innerhalb der Sterilisationskammer 3 auf. Jeder der Kunststoffvorformlinge 1 ist von Greifarmen 40 einer jeweiligen Greifklammer 41 umklammert. Dabei sind Klammergelenke 41 A der Greifklammern 41 entlang einer Bogenbahn auf der Transporteinrichtung 2 mechanisch fest fixiert. Im Ausführungsbeispiel der Figur 1A ist des Weiteren erkennbar, dass das Greifsystem 4 ein Verlagerungsmittel 5 zur Verlagerung von während des Sterilisierens innerhalb der Sterilisationskammer 3 durch das Greifsystem 4 vor einem Sterilisationsmedium abgeschatteten Kontaktflächen 1 A auf einer Außenfläche 1 B der Kunststoffvorformlinge 1 aufweist.

Dabei ist das Verlagerungsmittel 5 jeweils in Form einer Antriebsrolle 51 ausgebildet, welche drehbar innerhalb jeder Greifklammer 41 angeordnet ist und kraftschlüssig mit dem Kunststoffvorformling 1 in Verbindung steht. Dabei ist die Antriebsrolle 51 beispielsweise mittels eines pneumatischen, mechanischen, elektrischen oder hydraulischen Antriebes in Rotation versetzbar. Über eine Rotation der Antriebsrolle 51 um deren Rotationsachse, relativ zu den Greifarmen 40 der Greifklammer 41 in einer Drehrichtung, wird eine Rotation des Kunststoffvorformlings 1 um dessen Rotationsachse, relativ zu den Greifarmen 40 in die entgegengesetzte Richtung erzeugt.

Wie aus der Figur 1A erkennbar ist, dreht sich die Antriebsrolle 51 in Rechtsrichtung, während sich der Kunststoffvorformling 1 in Linksrichtung bewegt. Dabei werden relativ zur Außenfläche 1 B des Kunststoffvorformlings 1 zwischen dem Kunststoffvorformling 1 und den Greifarmen 40 der Greifklammern 41 auf der Außenfläche 1 B ausgebildete Kontaktflächen 1 A, welche für ein Sterilisationsmittel verdeckt sind, auf der Außenfläche 1 B des Kunststoffvorformlings 1 verlagert. Insofern werden während der Rotation die Kontaktflächen 1 A kontinuierlich auf der Außenfläche 1 B des Kunststoffvorformlings 1 verschoben, wodurch sämtliche Bereiche der Außenfläche 1 B des Kunststoffvorformlings 1 sterilisiert werden.

Das Bezugszeichen 8 kennzeichnet schematisch eine Strahlungseinrichtung, welche die Kunststoffvorformlinge zum Zwecke ihrer Sterilisation mit Elektronen beaufschlagt. Auch diese Beaufschlagung mit Elektronen bzw. allgemein Ladungsträgern wird im Rahmen der vorliegenden Anmeldung als Bestrahlung aufgefasst. Entsprechend wird auch ein Ladungsträgeremitter als Strahlungseinrichtung angesehen. Der besagte Reinraum, der die Kunststoffvorformlinge während ihrer Sterilisation umgibt kann dabei von einem stationären Gehäuse eingegrenzt sein. Es wäre jedoch auch möglich, dass der Reinraum den Transportpfad der Kunststoffvorformlinge ringkanalartig umgibt. Auch kann der Reinraum durch wenigstens eine stationäre und eine gegenüber dieser stationären Wand bewegliche Wand eingegrenzt sein, wobei bevorzugt zwischen dieser stationären Wand und der beweglichen Wand eine Dichtungseinrichtung, etwa in der Art eines sog. Wasserschlosses, angeordnet ist.

In der Figur 1B ist das in der Figur 1A gezeigte Greifsystem 4 sowie die Antriebsrolle 51 nochmals im Detail dargestellt.

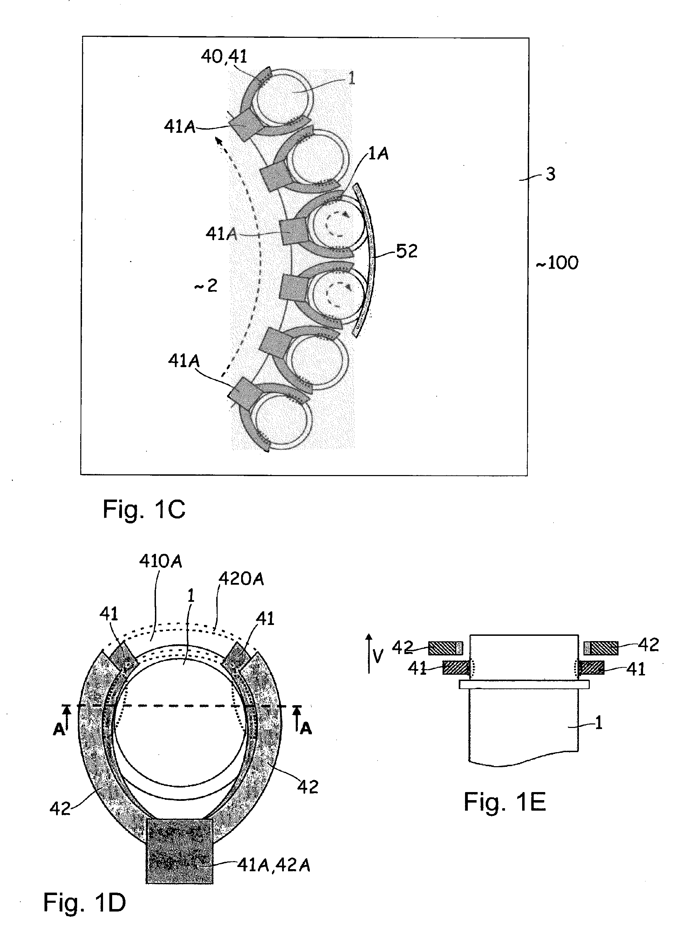

In der Figur 1C ist ein weiteres Ausführungsbeispiel der hier beschriebenen Vorrichtung 100 in einer schematischen Draufsicht gezeigt, bei dem anstatt der Antriebsrolle 51 eine Reibschiene 52 zur Rotation der Kunststoffvorformlinge 1 innerhalb der Greifklammern 41 während des Umgreifens und Tragens der Kunststoffvorformlinge 1 durch die Greifklammern 41 realisiert ist. Erkennbar ist, dass die Reibschiene 52 in horizontaler Richtung H den jeweiligen Klammergelenken 41A gegenüberliegend angeordnet ist. Werden die Kunststoffvorformlinge 1 nun durch die Transportvorrichtung 2 an der Reibschiene 52, das heißt an einer der Transporteinrichtung 2 zugewandten Außenfläche der Reibschiene 52 entlang- und vorbeigeführt, werden die Kunststoffvorformlinge 1 während des Zugreifens innerhalb der Greifklammern 41 in Rotation versetzt. Ein Verdrehwinkel der Kunststoffvorformlinge 1 ist daher unmittelbar durch eine Länge der Reibschiene 52 festgelegt. Mittels einer derartigen Reibschiene 52 ist es daher besonders einfach möglich, eine Verlagerung der Kontaktflächen 1 A zu bewerkstelligen.

In den Figuren 1D und 1E ist in einer schematischen Drauf-und Seitenansicht ein weiteres Ausführungsbeispiel einer hier beschriebenen Vorrichtung 100 gezeigt, bei denen im Unterschied zu den vorgenannten Ausführungsbeispielen das Verlagerungsmittel 5 eine weitere Greifklammer 42 aufweist. Beide Greifklammern 41, 42 sind in vertikaler Richtung V (siehe Figur 1 E) teilweise überlappend und in dieser vertikalen Richtung V beabstandet zueinander angeordnet. Während eines Sterilisierens ist zumindest eine der beiden Greifklammern 41, 42 in einer Zu-Stellung betrieben. Das heißt, dass in dieser Zu-Stellung eine der beiden Greifklammern den jeweiligen Kunststoffvorformling 1 umgreift und trägt. Bis zum Ablauf einer vorgebbaren Teilsterilisationszeit ist zum Beispiel die Greifklammer 41 in dieser Zu-Stellung während bis zu diesem Zeitpunkt die weitere Greifklammer 42 noch in der Offen-Stellung betrieben ist.

Zum Ablauf der Teilsterilisationszeit findet ein Umgreifen statt. Während dieses Übergabezeitbereiches sind beide Greifklammern 41, 42 in der Zu-Stellung. Nach Ablauf des Übergabezeitraumes verharrt nur die weitere Greifklammer 42 in der Zu-Stellung und die Greifklammer 41 wird nun in die Offen-Stellung gefahren. Mit anderen Worten wird der Kunststoffvorformling 1 von der Greifklammer 41 zur weiteren Greifklammer 42 übergeben, wobei während des Übergebens innerhalb der Sterilisationskammer 3 der Kunststoffvorformling 1 stets von einer der beiden Greifklammern 41, 42 festgehalten ist. Darüber hinaus ist erkennbar, dass sowohl das Klammergelenk 41 A der Greifklammer 41 als auch ein Klammergelenk 42A der weiteren Greifklammer 42 in vertikaler Richtung V übereinander angeordnet sind und jeweilige Klammeröffnungen 410A, 420A der beiden Greifklammern 41, 42 in der vertikalen Richtung V zumindest teilweise überlappen. In dieser Anordnung sind beide Greifklammern 41, 42 auf derselben Transporteinrichtung 2 mechanisch befestigt.

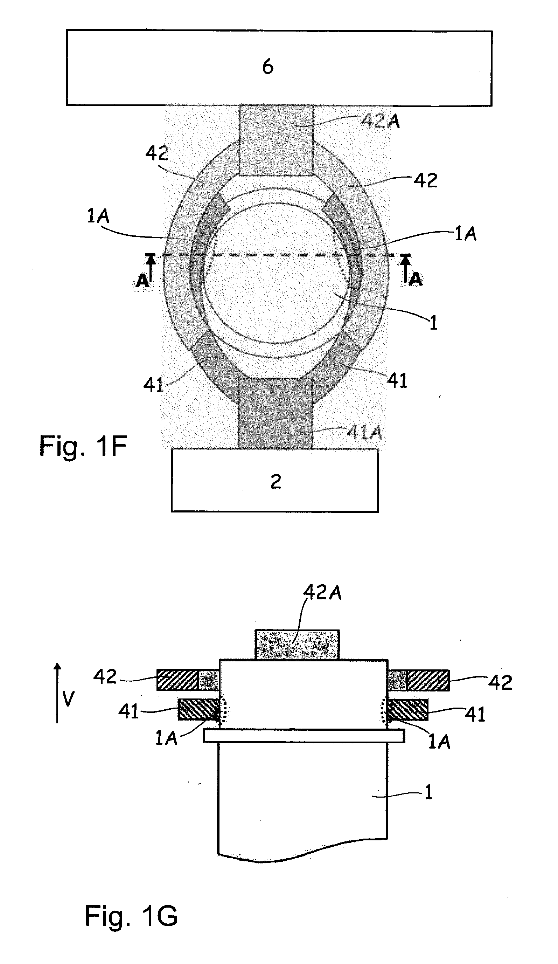

In den Figuren 1F und 1G ist ein weiteres Ausführungsbeispiel einer hier beschriebenen Vorrichtung 100 in einer schematischen Drauf-und Seitenansicht gezeigt. Im Unterschied zu der in den Figuren 1D und 1E gezeigten Vorrichtung 100 weist die Vorrichtung 100 der Figuren 1F und1G den Unterschied auf, dass die weitere Greifklammer 42 an einer weiteren Transporteinrichtung 6 und auf dieser mechanisch fest befestigt ist. Die Transporteinrichtungen 2 und 6 sind miteinander synchronisiert, insbesondere elektrisch oder mechanisch synchronisiert. Insofern liegen sich die Klammergelenke 41 A und die Klammergelenke 42A jeweils gegenüber. Daher können mittels der in diesem Ausführungsbeispiel beschriebenen Vorrichtung verschiedene Transporteinrichtungen miteinander kombiniert werden, um die Kontaktflächen 1 A der Kunststoffvorformlinge 1 während des Sterilisierens zu verlagern.

### Bezugszeichenliste

- V: vertikale Richtung
- H: horizontale Richtung
- 1: Kunststoffvorformling
- 1A: Kontaktflächen
- 1B: Außenfläche des Kunststoffvorformlings
- 2: Transporteinrichtung
- 3: Sterilisationskammer
- 4: Greifsystem
- 5: Verlagerungsmittel
- 6: weitere Transporteinrichtung
- 8: Strahlungseinrichtung
- 40: Greifarme
- 41, 42: Greifklammern
- 41 A, 41 B: Klammergelenke
- 410A, 410B: Klammeröffnungen
- 51: Antriebsrolle
- 52: Reibschiene
- 100: Vorrichtung
- 410A: Klammeröffnung
- 420A: Klammeröffnung

## Patentansprüche

1. Vorrichtung (100) zum Sterilisieren von Behältnissen (1) und insbesondere von Kunststoffvorformlingen (1), mit einer Sterilisationskammer (3) und einer zumindest teilweise innerhalb der Sterilisationskammer (3) angeordneten Transporteinrichtung (2) zum Bewegen der Kunststoffvorformlinge (1) innerhalb der Sterilisationskammer (3), wobei die Transporteinrichtung (2) zumindest ein Greifsystem (4) zum Umgreifen und Tragen der Kunststoffvorformlinge (1) mittels einer Greifklammer (41) aufweist, wobei
das Greifsystem (4) ein Verlagerungsmittel (5) zur Verlagerung während eines Sterilisierens innerhalb der Sterilisationskammer von durch das Greifsystem (4) vor einem Sterilisationsmedium und/oder einer Sterilisationsstrahlung abgeschatteten Kontaktflächen (1 A) auf einer Außenfläche (1 B) der Kunststoffvorformlinge (1) aufweist, **dadurch gekennzeichnet, dass**
das Verlagerungsmittel (5) mit zumindest einer weiteren Greifklammer (42) gebildet ist, wobei beide Greifklammern (41, 42) in vertikaler Richtung (V) zumindest teilweise überlappend und in dieser Richtung beanstandet zueinander angeordnet sind, und während des Sterilisierens zumindest eine Greifklammer in einer Offen-Stellung und eine davon verschiedene Greifklammer in einer Zu-Stellung betreibbar ist, und nach einer vorgebbaren Teil-Sterilisierungszeit die jeweils andere Greifklammer in einer Offen-Stellung oder einer Zu-Stellung betreibbar ist.

2. Vorrichtung (100) nach Anspruch 1,
**dadurch gekennzeichnet, dass**
das Verlagerungsmittel (5) mit zumindest einer Antriebsrolle (51) gebildet ist, welche drehbar innerhalb der Greifklammer (41) angeordnet ist und kraftschlüssig mit dem Kunststoffvorformling (1) in Verbindung steht, und wobei über eine Rotation der Antriebsrolle (51) um deren Rotationsachse, relativ zu Greifarmen (40) der Greifklammer (41) in eine Drehrichtung, eine Rotation des Kunststoffvorformlings (1) um dessen Rotationsachse, relativ zu den Greifarmen (40), bewirkbar ist, wobei beide Rotationsrichtungen jeweils entgegengesetzt sind.

3. Vorrichtung (100) nach Anspruch 1 oder 2,
**dadurch gekennzeichnet, dass**
die Antriebsrolle (51) mittels eines pneumatischen, mechanischen, elektrischen oder hydraulischen Antriebs rotierbar ist.

4. Vorrichtung (100) nach Anspruch 1,
**dadurch gekennzeichnet, dass**
das Verlagerungsmittel (5) mit zumindest einer Reibschiene (52) gebildet ist, wobei der Kunststoffvorformling (1) innerhalb der Sterilisationskammer (3) entlang dieser Reibschiene (52) vorbeiführbar ist, wobei über eine kraftschlüssige Verbindung zwischen dem Kunststoffvorformling (1) und der Reibschiene (52) der Kunststoffvorformling (1) innerhalb der Greifklammer um seine Rotationsachse drehbar ist.

5. Vorrichtung (100) nach Anspruch 1,
**dadurch gekennzeichnet, dass**
Klammergelenke (41A, 42A) der Greifklammern (41, 42) in vertikaler Richtung übereinander angeordnet sind, wobei jeweilige Klammeröffnungen (410A, 420A) der beiden Greifklammern (41, 42) in der vertikalen Richtung (V) zumindest teilweise überlappen.

6. Vorrichtung (100) nach Anspruch 1,
**dadurch gekennzeichnet, dass**
die Greifklammern (41, 42) an verschiedenen sich jeweils in horizontaler Richtung (H) gegenüberliegenden Klammergelenken (41A, 42A) angeordnet sind, wobei das Klammergelenk (42A) der weiteren Greifklammer (42) an einer weiteren Transporteinrichtung (6), welche zumindest teilweise innerhalb der Sterilisationskammer (3) angeordnet ist, befestigt ist.

7. Verfahren zum Sterilisieren von Behältnissen (1) und insbesondere von Kunststoffvorformlingen (1), bei dem über eine zumindest teilweise innerhalb einer Sterilisationskammer (3) angeordnete Transporteinrichtung (2) Kunststoffvorformlinge (1) bewegt werden, wobei die Transporteinrichtung (2) zumindest ein Greifsystem (4) zum Umgreifen und Tragen der Kunststoffvorformlinge (1) mittels einer Greifklammer (41) aufweist,
wobei
während eines Sterilisierens innerhalb der Sterilisationskammer, nach Ablauf einer vorgebbaren Teil-Sterilisationszeit, mittels eines Verlagerungsmittels (5), durch das Greifsystem (4) vor einem Sterilisationsmedium und/oder einer Sterilisationsstrahlung abgeschattete, Kontaktflächen (1A) der Kunststoffvorformlinge (1) verlagert werden, **dadurch gekennzeichnet, dass**
das Verlagerungsmittel (5) mit zumindest einer weiteren Greifklammer (42) gebildet wird, wobei beide Greifklammern (41, 42) in vertikaler Richtung (V) zumindest teilweise überlappend und in dieser Richtung beanstandet zueinander angeordnet sind, und während des Sterilisierens zumindest eine Greifklammer in einer Offen-Stellung und eine davon verschiedene Greifklammer in einer Zu-Stellung betrieben wird, und nach einer vorgebbaren Teil-Sterilisierungszeit die jeweils andere Greifklammer in einer Offen-Stellung oder einer Zu-Stellung betrieben wird.

8. Verfahren nach Anspruch 7,
**dadurch gekennzeichnet, dass**
das Behältnis (1) gegenüber dem es haltenden Greifsystem bezüglich einer Längsachse des Behältnisses (1) gedreht wird.

## Claims

1. Device (100) for sterilising containers (1) and in particular plastics material preforms (1), with a sterilisation chamber (3) and a transport device (2) disposed at least partially inside the sterilisation chamber (3) for moving the plastics material preforms (1) within the sterilisation chamber (3), wherein the transport device (2) has at least one gripping system (4) for engaging round and supporting the plastics material preforms (1) by means of a gripping clamp (41), wherein the gripping system (4) has a displacement means (5) for displacement during sterilisation within the sterilisation chamber of contact surfaces (1A) on an external surface (1B) of the plastics material preforms (1) which are shielded by the gripping system (4) from a sterilising medium and/or sterilising radiation,
**characterized in that**
the displacement means (5) is formed with at least one further gripping clamp (42), both gripping clamps (41, 42) being arranged in a vertical direction (V) at least partly in an overlapping manner and at a distance with respect to each other in this direction and wherein during sterilization at least one gripping clamp is able to be operated in an open position and a gripping clamp different thereto is able to be operated in a closed position and after a predefined partial sterilization time the respective different gripping clamp is able to be operated in an open or in a closed position.

2. Device (100) as claimed in Claim 1, **characterised in that** the displacement means (5) is formed with at least one drive roller (51) which is disposed rotatably within the gripping clamp (41) and is in non-positive engagement with the plastics material preforms (1), and wherein by means of a rotation of the drive roller (51) about its axis of rotation, relative to gripping arms (40) of the gripping clamp (41), in one direction of rotation, a rotation of the plastics material preform (1) around its axis of rotation, relative to the gripping arms (40), can be effected, wherein the two directions of rotation are in each case opposed.

3. Device (100) as claimed in Claim 1 or 2, **characterised in that** the drive roller (51) is rotatable by means of a pneumatic, mechanical, electrical or hydraulic drive.

4. Device (100) as claimed in Claim 1, **characterised in that** the displacement means (5) is formed with at least one friction (52) rail, wherein the plastics material preform (1) can be guided along this friction rail (52) within the sterilisation chamber (3), wherein by means of a non-positive engagement between the plastics material preform (1) and the friction rail (52) the plastics material preform (1) is rotatable about its axis of rotation within the gripping clamp.

5. Device (100) as claimed in Claim 1, **characterised in that** clamp joints (41 A, 42A) of the gripping clamps (41, 42) are disposed above one another in the vertical direction, wherein respective clamp openings (410A, 420A) of the two gripping clamps (41, 42) overlap at least partially in the vertical direction (V).

6. Device (100) as claimed in Claim 1, **characterised in that** the gripping clamps (41, 42) are disposed on different clamp joints (41 A, 42A) which each lie opposite one another in the horizontal direction (H), wherein the clamp joint (42A) of the further gripping clamp (42) is fastened on a further transport device (6) which is disposed at least partially within the sterilisation chamber (3).

7. Method for sterilising containers (1) and in particular plastics material preforms (1), in which plastics material preforms (1) are moved by means of a transport device (2) disposed at least partially inside a sterilisation chamber (3), wherein the transport device (2) has at least one gripping system (4) for engaging round and supporting the plastics material preforms (1) by means of a gripping clamp (41), wherein, during sterilisation within the sterilisation chamber, after expiry of a predeterminable partial sterilisation time contact surfaces (1A) of the plastics material preforms (1) which are shielded by the gripping system (4) from a sterilising medium and/or sterilising radiation are displaced by a displacement means (5),
**characterized in that**
the displacement means (5) is formed with at least one further gripping clamp (42), both gripping clamps (41, 42) being arranged in a vertical direction (V) at least partly in an overlapping manner and at a distance with respect to each other in this direction and at least one gripping clamp is operated in an open position and a gripping clamp different thereto is operated in a closed position and after a predefinable partial sterilization time the respective different gripping clamp is operated in an open or in a closed position.

8. Method as claimed in Claim 7, **characterised in that** the container (1) is rotated relative to the gripping system holding it with respect to a longitudinal axis of the container (1).

## Revendications

1. Installation (100) pour la stérilisation de récipients (1) et en particulier de préformes en matière plastique (1), comprenant un compartiment de stérilisation (3) et un dispositif de transport (2) disposé au moins en partie à l'intérieur du compartiment de stérilisation (3) pour le déplacement des préformes en matière plastique (1) à l'intérieur du compartiment de stérilisation (3), ledit dispositif de transport (2) comportant au moins un système de préhension (4) pour saisir et supporter les préformes en matière plastique (1) au moyen d'une pince de préhension (41),
le système de préhension (4) comprenant un moyen de déplacement (5), destiné à amener devant un fluide de stérilisation et/ou un rayonnement de stérilisation des surfaces de contact (1 A) sur une surface extérieure (1 B) des préformes en matière plastique (1) cachées par le système de préhension (4), lors d'une stérilisation à l'intérieur du compartiment de stérilisation,
**caractérisée en ce que**
le moyen de déplacement (5) est formé avec au moins une autre pince de préhension (42), les deux pinces de préhension (41, 42) se chevauchant au moins en partie dans la direction verticale (V) et étant espacées l'une de l'autre dans cette direction, et au moins une pince de préhension pouvant être commandée dans une position d'ouverture et une pince de préhension différente de la précédente dans une position de fermeture lors de la stérilisation, et l'autre pince de préhension pouvant être commandée dans une position d'ouverture ou une position de fermeture après un temps de stérilisation partiel définissable.

2. Installation (100) selon la revendication 1,
**caractérisée en ce que**
le moyen de déplacement (5) est formé avec au moins un galet d'entraînement (51) disposé de manière à être rotatif à l'intérieur de la pince de préhension (41) et relié par adhérence à la préforme en matière plastique (1), et **en ce que**, par rotation dans un sens de rotation du galet d'entraînement (51) autour de son axe de rotation par rapport aux bras (40) de la pince de préhension (41), une rotation de la préforme en matière plastique (1) est obtenue autour de son axe de rotation par rapport aux bras de préhension (40), les deux sens de rotation étant opposés.

3. Installation (100) selon la revendication 1 ou 2,
**caractérisée en ce que**
le galet d'entraînement (51) peut être entraîné en rotation au moyen d'un entraînement pneumatique, mécanique, électrique ou hydraulique.

4. Installation (100) selon la revendication 1,
**caractérisée en ce que**
le moyen de déplacement (5) est formé avec au moins un rail de friction (52), la préforme en matière plastique (1) pouvant être translatée le long dudit rail de friction (52) à l'intérieur du compartiment de stérilisation (3), la préforme en matière plastique (1) étant rotative autour de son axe de rotation à l'intérieur de la pince de préhension au moyen d'une liaison par adhérence entre la préforme en matière plastique (1) et le rail de friction (52).

5. Installation (100) selon la revendication 1,
**caractérisée en ce que**
des articulations (41 A, 42A) des pinces de préhension (41, 42) sont superposées dans la direction verticale, les ouvertures (410A, 420A) respectives des deux pinces de préhension (41, 42) se chevauchant au moins en partie dans la direction verticale (V).

6. Installation (100) selon la revendication 1,
**caractérisée en ce que**
les pinces de préhension (41, 42) sont disposées contre différentes articulations (41 A, 42A) en vis-à-vis dans la direction horizontale (H), l'articulation (42A) de l'autre pince de préhension (42) étant fixée contre un autre dispositif de transport (6), lequel est au moins en partie disposé à l'intérieur du compartiment de stérilisation (3).

7. Procédé de stérilisation de récipients (1) et en particulier de préformes en matière plastique (1), dans lequel des préformes en matière plastique (1) sont déplacées au moyen d'un dispositif de transport (2) disposé au moins en partie à l'intérieur d'un compartiment de stérilisation (3), ledit dispositif de transport (2) comportant au moins un système de préhension (4) pour saisir et supporter les préformes en matière plastique (1) au moyen d'une pince de préhension (41), dans lequel,
lors d'une stérilisation à l'intérieur du compartiment de stérilisation, après un temps de stérilisation partiel définissable, des surfaces de contact (1 A) des préformes en matière plastique (1) cachées par le système de préhension (4) sont amenées par un moyen de déplacement (5) devant un fluide de stérilisation et/ou un rayonnement de stérilisation,
**caractérisé en ce que**
le moyen de déplacement (5) est formé avec au moins une autre pince de préhension (42), les deux pinces de préhension (41, 42) se chevauchant au moins en partie dans la direction verticale (V) et étant espacées l'une de l'autre dans cette direction, et au moins une pince de préhension étant commandée dans une position d'ouverture et une pince de préhension différente de la précédente dans une position de fermeture lors de la stérilisation, et l'autre pince de préhension étant commandée dans une position d'ouverture ou une position de fermeture après un temps de stérilisation partiel définissable.

8. Procédé selon la revendication 7,
**caractérisé en ce que**
le récipient (1) est tourné vis-à-vis du système de préhension qui le maintient par rapport à un axe longitudinal du récipient (1).
